# EUROPEAN PATENT APPLICATION

(11) **EP 4 160 610 A1**
(43) Date of publication of application: **05.04.2023**
(21) Application number: 21199652.5
(22) Date of filing: 29.09.2021
(51) Int. Cl.: G16H 40/20, G16H 40/67, G07C 1/10, G06Q 10/06, H04W 4/02, G01S 19/42

(54) **SYSTEM AND METHOD FOR RECORDING ATTENDANCE OF A CAREGIVER**

(71) Applicant: Chao, Shih-Chang, Taipei City Sinyi Dist. 110 (TW)
(72) Inventor: Chao, Shih-Chang, Taipei City Sinyi Dist. 110 (TW)
(74) Representative: Style, Kelda Camilla Karen

(57) **Abstract**

A system includes a client device (2), a caregiver device (3) and a server (1). The server (1) transmits an address of a record for a care session to a digital map provider (8) that converts the address into caregiving coordinates and transmits the caregiving coordinates. The caregiver device (3) obtains interface data from the server (1) and displays an interface based on the interface data for allowing a caregiver to sign in with respect to the care session. When a button of the interface is operated, the server (1) enables a geolocation service provider (9) to request position data from the caregiver device (3), and to transmit sign-in coordinates to the server (1) with reference to the position data. The client device (2) displays the caregiving coordinates together with the sign-in coordinates.

## Description

The disclosure relates to a system and a method for recording attendance of a caregiver.

Conventionally, when someone (client) needs care service to care for, for example, an elderly person, an infant or a child at a caregiving location, he/she may make an appointment through a matching platform that provides matching service to match a caregiver with the requirement of the care service. The caregiver can operate an application provided by the matching platform to sign in when arriving at the caregiving location, and to sign out when the care service has ended.

Therefore, an object of the disclosure is to provide a system and a method for recording attendance of a caregiver.

According to one aspect of the disclosure, a system for recording attendance of a caregiver who is expected to provide a care session of care service at a caregiving location according to an appointment made by a client is provided. The system includes a server, a client device and a caregiver device.

The server is configured to communicate with a digital map provider and a geolocation service provider, to store an appointment record that is related to the appointment and that has an address corresponding to the caregiving location, and to transmit the address to the digital map provider for the digital map provider to convert the address into a set of caregiving coordinates in a geographic coordinate system (GCS) and to transmit the set of caregiving coordinates to the server.

The client device is adapted to be possessed by the client, and is configured to communicate with the server, to request, from the server, the set of caregiving coordinates corresponding to the caregiving location, and to display the set of caregiving coordinates.

The caregiver device is adapted to be possessed by the caregiver, and is configured to communicate with the geolocation service provider, to communicate with the server to obtain from the server interface data that is related to an attendance interface containing a sign-in button, to display the attendance interface based on the interface data so as to allow the caregiver to sign in with respect to the care session by operating the sign-in button.

The server is further configured to, when it is determined that the sign-in button of the attendance interface is operated, enable the geolocation service provider to request sign-in position data related to a first current geographic position of the caregiver device from the caregiver device, and to transmit, to the server with reference to the sign-in position data, a set of sign-in coordinates corresponding to the first current geographic position of the caregiver device in the GCS.

The client device is further configured to request the set of sign-in coordinates from the server, and to display the set of sign-in coordinates together with the set of caregiving coordinates so as to allow the client to determine whether the caregiver signed in at the caregiving location.

According to another aspect of the disclosure, a method for recording attendance of a caregiver who is expected to provide care service at a caregiving location according to an appointment made by a client is provided. The method is to be implemented by a server communicating with a client device, a caregiver device, a digital map provider and a geolocation service provider. The server stores an appointment record that is related to the appointment and that has an address corresponding to the caregiving location. The client device is possessed by the client. The caregiver device is possessed by the caregiver and is capable of communicating with the geolocation service provider. The method includes steps of:
transmitting the address to the digital map provider for the digital map provider to convert the address into a set of caregiving coordinates in a geographic coordinate system (GCS) and to transmit the set of caregiving coordinates to the server;
in response to a request from the client device for the set of caregiving coordinates, transmitting the set of caregiving coordinates corresponding to the caregiving location to the client device for the client device to display the set of caregiving coordinates;
transmitting, to the caregiver device, interface data that is related to an attendance interface containing a sign-in button for the caregiver device to display the attendance interface based on the interface data so as to allow the caregiver to sign in with respect to the care session by operating the sign-in button;
when it is determined that the sign-in button of the attendance interface is operated, enabling the geolocation service provider to request sign-in position data related to a first current geographic position of the caregiver device from the caregiver device, and to transmit, to the server with reference to the sign-in position data, a set of sign-in coordinates corresponding to the first current geographic position of the caregiver terminal in the GCS; and
in response to a request from the client device for the set of sign-in coordinates, transmitting the set of sign-in coordinates to the client device for the client device to display the set of sign-in coordinates together with the set of caregiving coordinates so as to allow the client to determine whether the caregiver signed in at the caregiving location.

Other features and advantages of the disclosure will become apparent in the following detailed description of the embodiment with reference to the accompanying drawings, of which:
Figure 1 is a block diagram illustrating an embodiment of a system for recording attendance of a caregiver according to the disclosure;
Figure 2 is a schematic diagram illustrating an operation display of an application program executed by a client device of the system according to an embodiment of the disclosure;
Figure 3 is a schematic diagram illustrating an operation display displayed by the client device or a caregiver device of the system according to an embodiment of the disclosure;
Figure 4 is a schematic diagram illustrating an attendance interface displayed by the caregiver device according to an embodiment of the disclosure;
Figure 5 is a schematic diagram illustrating an authorization interface displayed by the caregiver device according to an embodiment of the disclosure;
Figure 6 is a schematic diagram illustrating an entry of attendance history that includes each sign-in time and each sign-out time for each care session of care service according to an embodiment of the disclosure;
Figures 7 and 8 are schematic diagrams illustrating different views of a digital map provided by a digital map provider; and
Figures 9A to 9C are flow charts cooperatively illustrating a method for recording attendance of a caregiver according to an embodiment of the disclosure.

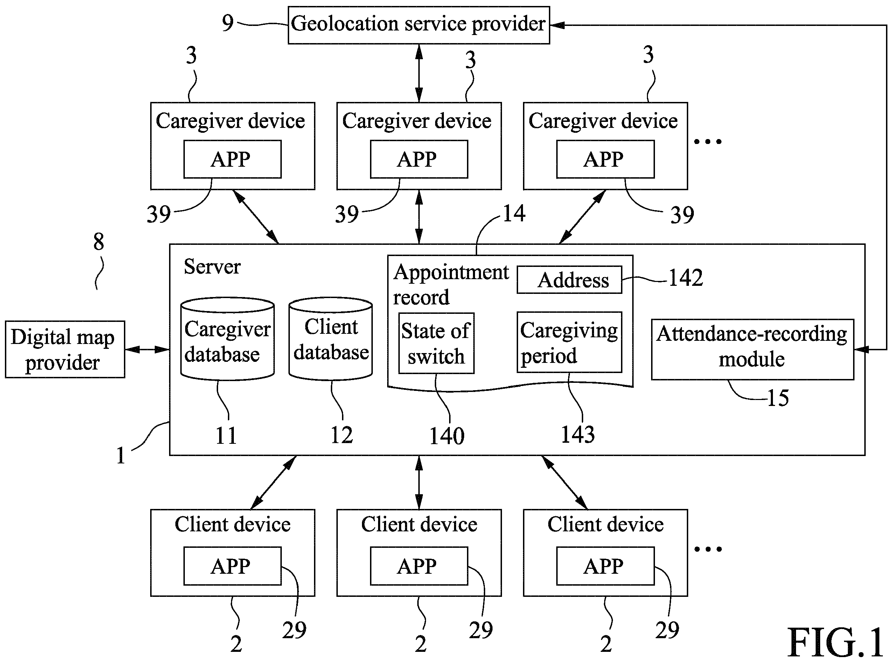

Referring to Figure 1, an embodiment of a system for recording attendance of a caregiver is illustrated. The caregiver is expected to provide a care session of care service at a caregiving location within a caregiving period according to an appointment made by a client with the caregiver. The appointment may include a single care session or multiple care sessions. Each care session may be part of a long-term care for caring an elderly person, a one-time service for caring an elderly person, part of a long-term babysitting service for caring an infant or a child, a one-time babysitting service, or the like. The caregiver may be a nursing personnel or a babysitter. The client may be related to a person who demands and is going to receive the care service (i.e., the care receiver) . For example, the client and the care receiver are family.

The system includes a server 1, a plurality of client devices 2 and a plurality of caregiver devices 3.

The server 1 is configured to communicate with a digital map provider 8 and a geolocation service provider 9. Each of the digital map provider 8 and the geolocation service provider 9 is a server providing web map service such as Google Maps and Apple Maps, but is not limited thereto.

The server 1 includes a caregiver database 11, a client database 12 and an attendance-recording module 15. The server 1 fulfills functions described in this disclosure under the assistance of the attendance-recording module 15.

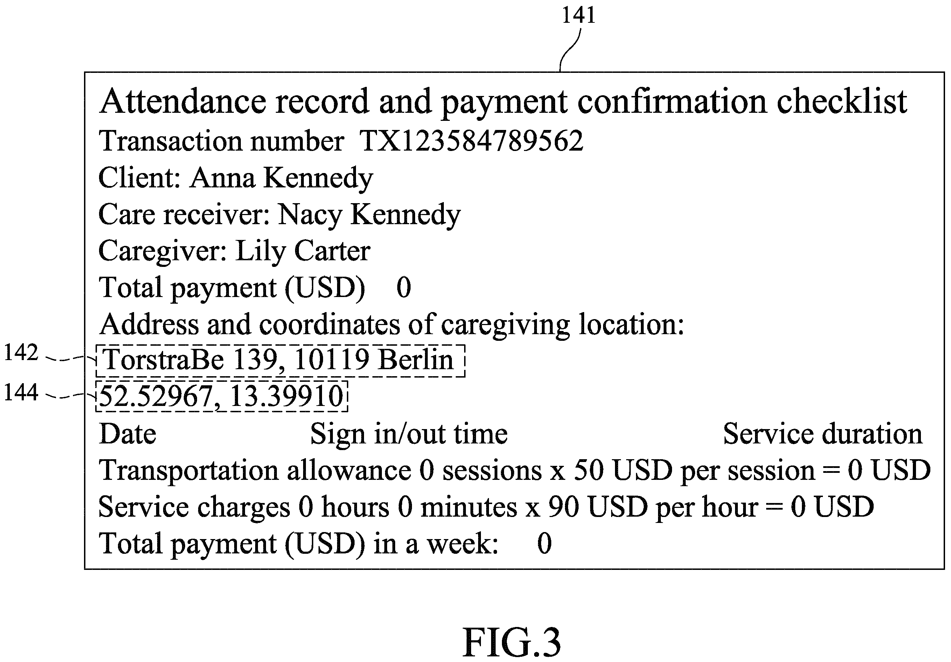

The server 1 is configured to store an appointment record 14 that is related to the appointment made between the caregiver and the client. The appointment record 14 has a current state of a switch 140, an entry of the caregiving period 143, and an address 142 corresponding to the caregiving location. The server 1 is configured to transmit the address 142 to the digital map provider 8 for the digital map provider 8 to convert the address 142 into a set of caregiving coordinates in a geographic coordinate system (GCS), and then the digital map provider 8 transmits the set of caregiving coordinates to the server 1. Figure 3 illustrates an operation screen that is displayed by the client device 2 or the caregiver device 3 of the system and that shows the set of caregiving coordinates 144 according to an embodiment.

In this embodiment, the attendance-recording module 15 may be implemented by one of hardware, firmware, software, and any combination thereof. For example, the attendance-recording module 15 may be implemented to be software modules in a program, where the software modules contain codes and instructions to carry out specific functionalities, and can be called individually or together to fulfill functions of the attendance-recording module 15 of this disclosure. The above-mentioned modules may be embodied in: executable software as a set of logic instructions stored in a machine- or computer-readable storage medium of a memory such as random access memory (RAM), read only memory (ROM) , programmable ROM (PROM), firmware, flash memory, etc.; configurable logic such as programmable logic arrays (PLAs), field programmable gate arrays (FPGAs), complex programmable logic devices (CPLDs), etc.; fixed-functionality logic hardware using circuit technology such as application specific integrated circuit (ASIC), complementary metal oxide semiconductor (CMOS), transistor-transistor logic (TTL) technology, etc.; or any combination thereof.

Each client device 2 is possessed by a corresponding client, and is configured to store an application program (APP) 29 and to execute the APP 29 to communicate with the server 1. A possessor of the client device 2 can operate the APP 29 to register as a client of the care service, and then the APP 29 allows the client to request the care service, to make payment for the care service, to designate the current state of the switch 140, and to check attendance history of the caregiver. The client device 2 may be a smartphone, a personal computer, a tablet computer, etc., but implementation of the client device 2 is not limited to the disclosure herein and may vary in other embodiments.

Each caregiver device 3 is possessed by a corresponding caregiver, and is configured to store an APP 39 and to execute the APP 39 to communicate with the server 1. A possessor of the caregiver device 3 can operate the APP 39 to register as a caregiver for the care service, and then the APP 39 allows the caregiver to submit to the server 1 a cost estimate for the care service requested by the client, to sign in or sign out for each care session upon arrival or departure, and to check his/her attendance history. The caregiver device 3 may be a smartphone or a tablet computer capable of positioning, but implementation of the caregiver device 3 is not limited to the disclosure herein and may vary in other embodiments.

Since all client devices 2 operate in the same fashion, and since all caregiver devices 3 operate in the same fashion, the following description will be given with respect to a single client device 2 and a single caregiver device 3 for the sake of simplicity.

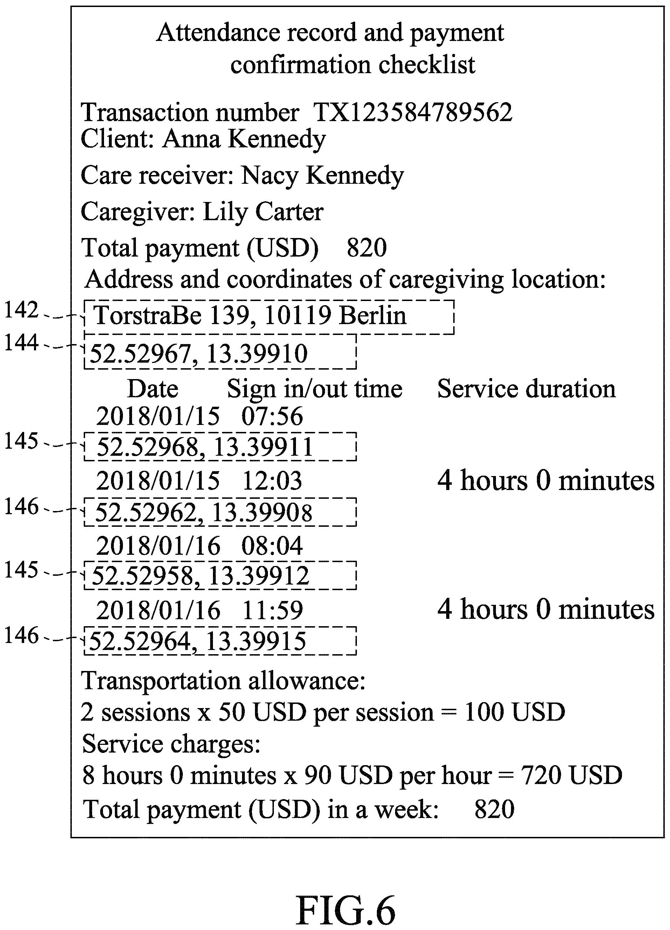

More specifically, the client device 2 is configured to be operated by the client to make payment for the care service (e.g., for specific care session(s), or the entire appointment including one or multiple care sessions), and the server 1 is configured to, when it is determined that the payment for the care service has been made, transmit the address 142 to the digital map provider 8 for the digital map provider 8 to convert the address 142 into the set of caregiving coordinates 144 and transmit the set of caregiving coordinates 144 to the server 1. Then, the client device 2 is configured to communicate with the server 1, to request from the server 1 the set of caregiving coordinates 144 corresponding to the caregiving location, and to display the set of caregiving coordinates 144 as shown in Figures 3 and 6. It should be noted that the caregiver device 3 is also capable of requesting from the server 1 the set of caregiving coordinates 144 corresponding to the caregiving location, and displaying the set of caregiving coordinates 144.

The client device 2 is further configured to be operated by the client operating the APP 29 to switch the current state of the switch 140 of the appointment record 14 stored in the server 1 between an ON state and an OFF state. For example, the appointment record 14 is a data file having a bit field, a flag, or the like for storing the current state of the switch 140. The ON state indicates that attendance registration (namely, the act of signing in or out) by the caregiver device 3 is allowed; the OFF state indicates that attendance registration by the caregiver device 3 is not allowed.

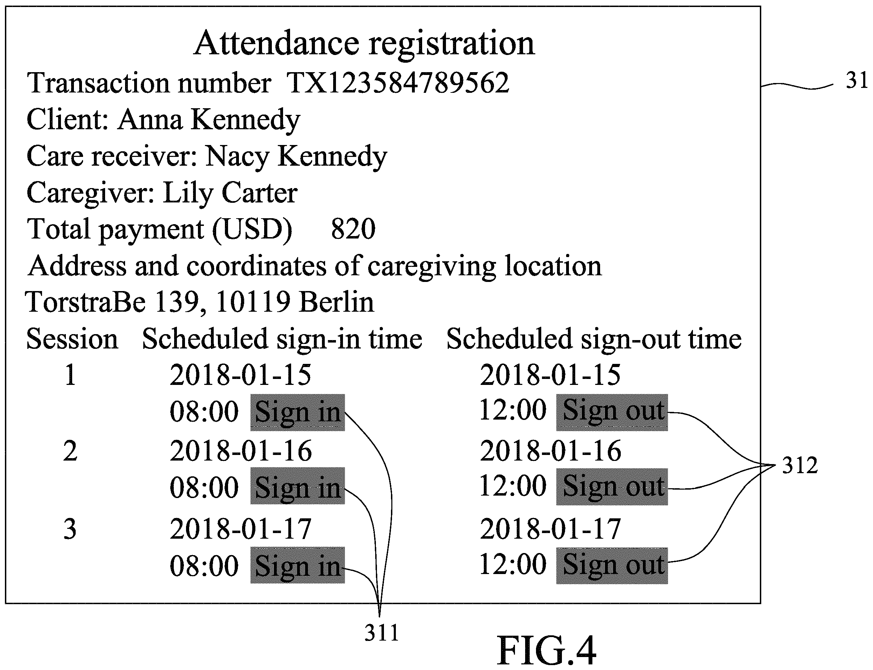

The server 1 is configured to provide interface data that is related to an attendance interface 31 (see Figure 4) to the caregiver device 3 so as to allow the caregiver device 3 to display the attendance interface 31 for allowing the caregiver to sign in and sign out. In this embodiment, the attendance interface 31 contains a plurality of sign-in buttons 311 and a plurality of sign-out buttons 312 corresponding respectively to the sign-in buttons 311. Each of the sign-in buttons 311 and the corresponding one of the sign-out buttons 312 are used respectively to sign in and sign out with respect to a single care session of care service (e.g., with the caregiving period being from 08:00 to 12:00 on January 15, 2018). In particular, the caregiver is allowed to sign in with respect to a care session by operating the sign-in button 311, and to sign out with respect to the care session by operating the sign-out button 312. For example, the caregiver device 3 may include a touch screen for displaying the attendance interface 31, and the caregiver can operate each of the sign-in buttons 311 and the sign-out buttons 312 by touching a position of the touch screen that corresponds thereto.

The following description relating to signing in and out of the caregiver will be made with respect to a single care session (and the corresponding sign-in and sign-out buttons 311, 312).

The caregiver device 3 is configured to communicate with the geolocation service provider 9, to communicate with the server 1 to obtain from the server 1 the interface data, and to display the attendance interface 31 based on the interface data for allowing the caregiver to sign in and sign out when it is determined that the current state of the switch 140 is in the ON state and that a current time is within a preset period, for example, 30 minutes before a start time point of the caregiving period 143.

The caregiver device 3 is configured to, when it is determined that the sign-in button 311 is operated, record a time instant when the sign-in button 311 is being operated (referred to as a "first time instant") as a sign-in time for thee care session, and transmit the sign-in time to the server 1 for storage of the sign-in time in the server 1.

The server 1 is further configured to, when it is determined that the sign-in button 311 of the attendance interface 31 is operated, enable the geolocation service provider 9 to request sign-in position data related to a first current geographic position of the caregiver device 3 from the caregiver device 3 (the current geographic position of the caregiver device 3 at the time of operating the sign-in button 311 is referred to as a "first current geographic position" of the caregiver device 3), and to transmit, to the server 1 with reference to the sign-in position data, a set of sign-in coordinates 145 (see Figure 6) that corresponds to the first current geographic position of the caregiver device 3 in the GCS. It should be noted that the sign-in position data includes information related to altitude, longitude and latitude, and the set of sign-in coordinates 145 merely includes information related to longitude and latitude.

The client device 2 is further configured to request the set of sign-in coordinates 145 from the server 1, and to display the set of sign-in coordinates 145 together with the set of caregiving coordinates 144 so as to allow the client to determine whether the caregiver signed in at the caregiving location.

Similarly, the caregiver device 3 is further configured to, when it is determined that the sign-out button 312 is operated, record a time instant when the sign-out button 312 is being operated (referred to as a "second time instant") as a sign-out time for the care session, and transmit the sign-out time to the server 1 for storage of the sign-out time in the server 1.

The server 1 is further configured to, when it is determined that the sign-out button 312 of the attendance interface 31 is operated, enable the geolocation service provider 9 to request sign-out position data related to a current geographic position of the caregiver device 3 from the caregiver device 3 (the current geographic position of the caregiver device 3 at the time of operating the sign-out button 312 is referred to as a "second current geographic position" of the caregiver device 3), and to transmit, to the server 1 with reference to the sign-out position data, a set of sign-out coordinates 146 corresponding to the second current geographic position of the caregiver device 3 in the GCS. Similarly, the sign-out position data includes information related to altitude, longitude and latitude, and the set of sign-out coordinates 146 merely includes information related to longitude and latitude.

The client device 2 is further configured to request the set of sign-out coordinates 146 from the server 1, and to display the set of sign-out coordinates 146 together with the set of caregiving coordinates 144 so as to allow the client to determine whether the caregiver signed out at the caregiving location.

Additionally, the server 1 is configured, with respect to the appointment, to record an entry of attendance history that includes the sign-in time and the sign-out time for each care session of the appointment and that corresponds to the appointment record 14. Each of the client device 2 and the caregiver device 3 is further configured to obtain the entry of attendance history corresponding to the appointment record 14 from the server 1 and to display the entry of attendance history thus obtained.

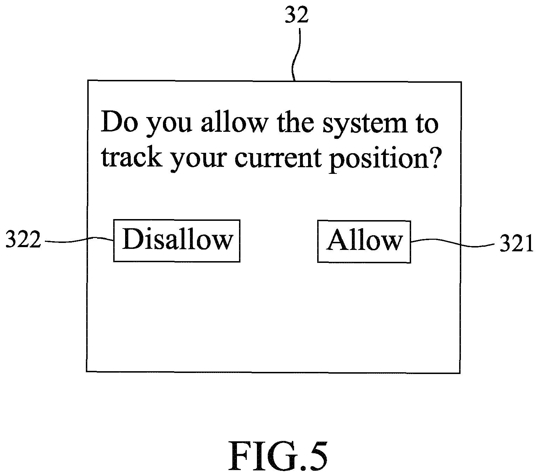

Furthermore, the server 1 is further configured to, when it is determined that the sign-in button 311 is operated on the caregiver device 3 for the first time with respect to the care session, allow the caregiver device 3 to display an authorization interface 32 as shown in Figure 5 to ask the caregiver whether to allow the server 1 to obtain the set of sign-in coordinates 145 or not. The authorization interface 32 contains an allowing button 321 and a disallowing button 322. The allowing button 321 indicates that the caregiver allows the server 1 to obtain a current geographic position of the caregiver device 3. The disallowing button 322 indicates that the caregiver does not allow the server 1 to obtain the current geographic position of the caregiver device 3. When it is determined that the allowing button 321 is operated on the caregiver device 3, the server 1 is configured to transmit a request for the set of sign-in coordinates 145 to the geolocation service provider 9 for the geolocation service provider 9 to request the sign-in position data related to the current geographic position of the caregiver device 3 from the caregiver device 3 and to transmit, to the server 1 with reference to the sign-in position data, the set of sign-in coordinates 145, thereby obtaining the set of sign-in coordinates 145 from the geolocation service provider 9. On the other hand, when it is determined that the disallowing button 322 is operated on the caregiver device 3, the server 1 does not transmit the request for the set of sign-in coordinates 145 to the geolocation service provider 9, thereby not obtaining the set of sign-in coordinates 145 from the geolocation service provider 9. Additionally, after it is determined that the disallowing button 322 is operated on the caregiver device 3, the server 1 is configured to enable the caregiver device 3 to display the authorization interface 32 again to ask the caregiver whether to allow the server 1 to obtain the current geographic position of the caregiver device 3 or not when it is determined that the sign-out button 312 is operated on the caregiver device 3 or that the sign-in button 311 is operated again.

It is worth to note that the set of caregiving coordinates 144 corresponding to the caregiving location, the set of sign-in coordinates 145 corresponding to the first current geographic position of the caregiver device 3, and the set of sign-out coordinates 146 corresponding to the second current geographic position of the caregiver device 3 are each a set of horizontal coordinates that contains longitude and latitude each being measured in decimal degrees and having five decimal places. For example, for an address corresponding to a caregiving location "TorstraBe 139, 10119 Berlin", the set of caregiving coordinates 144 corresponding thereto is "52.52967, 13.39910".

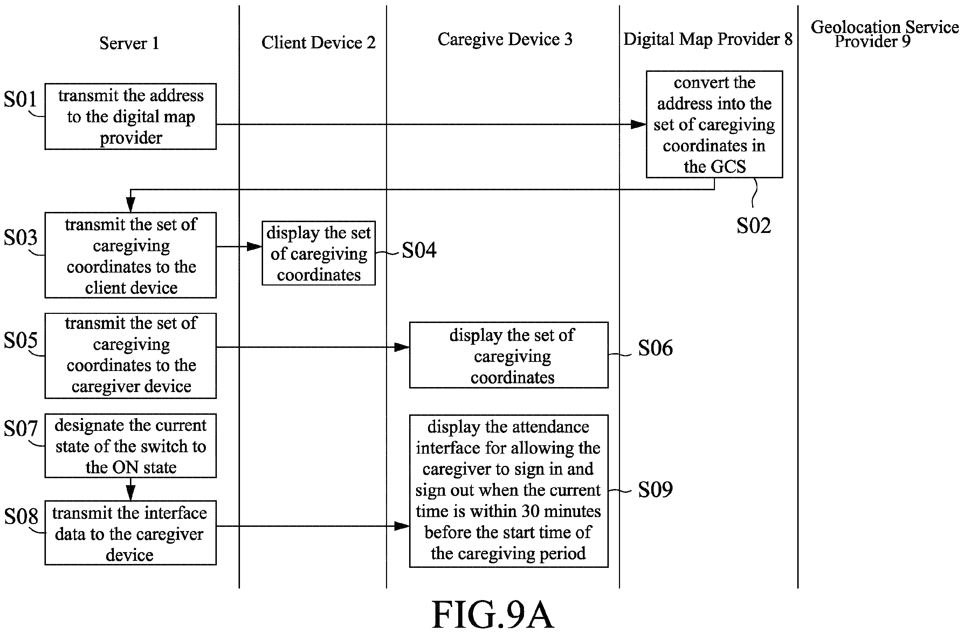

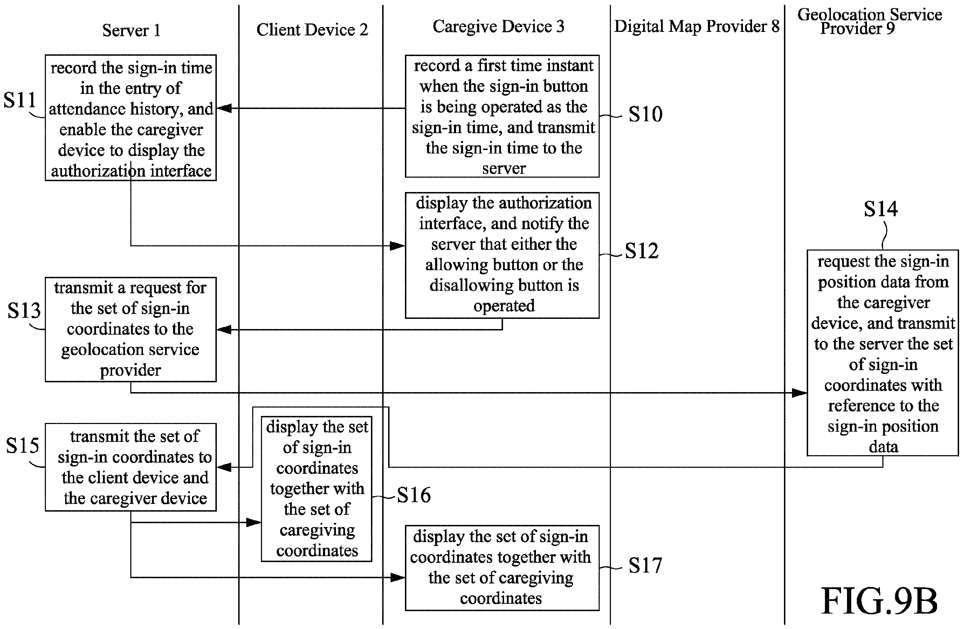

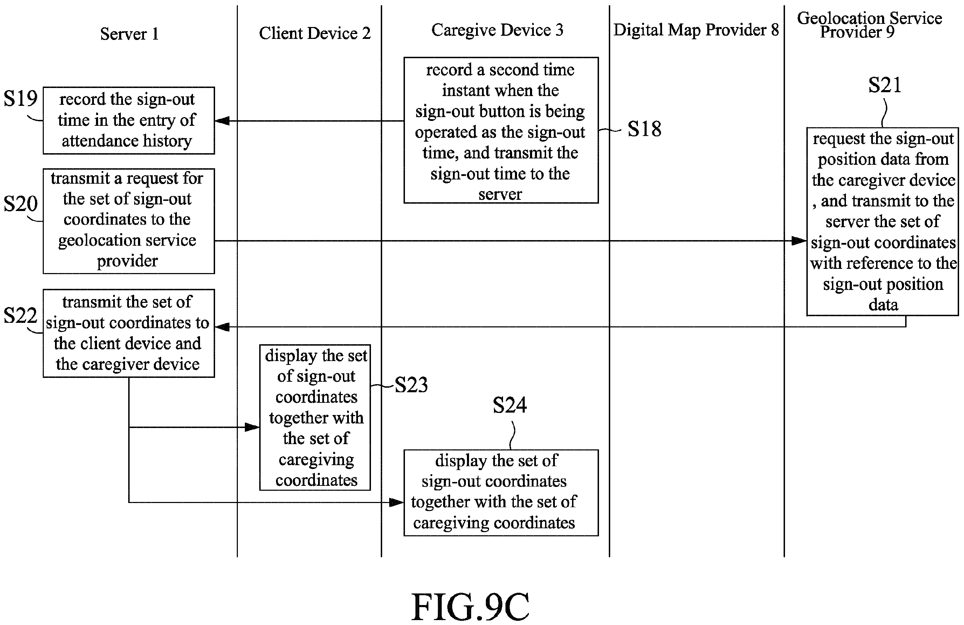

Referring to Figures 9A to 9C, an embodiment of a method for recording attendance of a caregiver according to the disclosure is illustrated. The method is to be implemented by the system according to the disclosure that is previously described.

In step S01, the server 1 transmits the address 142 to the digital map provider 8 when it is determined that the payment for the care service has been made. For example, the client may operate the APP 29 executed by the client device 2 to make the payment for the care service via a bank server (not shown) operated by a bank, and the bank server is configured to transmit a message to the server 1 to notify the server of completion of the payment for the care service upon receiving the payment.

In step S02, upon receiving the address 142, the digital map provider 8 converts the address 142 into the set of caregiving coordinates 144 in the GCS, and transmits the set of caregiving coordinates 144 to the server 1.

In response to a request from the client device 2 for the set of caregiving coordinates 144, the server 1 transmits the set of caregiving coordinates 144 to the client device 2 in step S03, and then the client device 2 displays the set of caregiving coordinates 144 in step S04 (see Figure 3).

Similarly, in response to a request from the caregiver device 3 for the set of caregiving coordinates 144, the server 1 transmits the set of caregiving coordinates 144 to the caregiver device 3 in step S05, and then the caregiver device 3 displays the set of caregiving coordinates 144 in step S06 (see Figure 3). It should be noted that steps S03 and S05 are independently executed.

In step S07, the server 1 designates the current state of the switch 140 to the ON state when it is determined that the payment for the care service has been made.

In step S08, the server 1 transmits the interface data to the caregiver device 3 so as to allow the caregiver device 3 to display the attendance interface 31 when the server 1 determines that the current state of the switch 140 is the ON state. Then, in step S09, the caregiver device 3 determines whether a current time is within the preset period (e.g., 30 minutes) before the start time point of the caregiving period 143, and displays the attendance interface 31 based on the interface data for allowing the caregiver to sign in and sign out when it is determined that the current time is within the preset period (e.g., 30 minutes) before the start time of the caregiving period 143.

Steps S10 to S18 are implemented when the caregiver operates the sign-in button 311 on the caregiver device 3.

In step S10, when it is determined by the caregiver device 3 that the sign-in button 311 is operated, the caregiver device 3 records a time instant when the sign-in button 311 is being operated (a first time instant) as the sign-in time, and transmits the sign-in time to the server 1 for storage of the sign-in time in the server 1. In some embodiments, when the sign-in button 311 is operated, the caregiver device 3 further communicates with the server 1 through the APP 39 to notify the server 1 that the sign-in button 311 is operated.

In step S11, the server 1 records the sign-in time in the entry of attendance history. Further, when it is determined that the sign-in button 311 is operated on the caregiver device 3 for the first time with respect to the care session, the server 1 enables the caregiver device 3 to display the authorization interface 32 (Figure 5) to ask the caregiver whether to allow the server 1 to obtain the set of sign-in coordinates 145 or not. In step S12, the caregiver device 3 displays the authorization interface 32, and notifies the server 1 that either the allowing button 321 or the disallowing button 322 is operated when one of the allowing button 321 and the disallowing button 322 is operated.

When it is determined that the allowing button 321 is operated on the caregiver device 3, the server 1 transmits a request for the set of sign-in coordinates 145 to the geolocation service provider 9 in step S13. Then, in step S14, the geolocation service provider 9 requests for the sign-in position data from the caregiver device 3 in response to receipt of the request for the set of sign-in coordinates 145 from the server 1, and transmits to the server 1 the set of sign-in coordinates 145 with reference to the sign-in position data. Accordingly, the server 1 obtains the set of sign-in coordinates 145 from the geolocation service provider 9. In response to a request from the client device 2 for the set of sign-in coordinates 145, the server 1 transmits the set of sign-in coordinates 145 to the client device 2 in step S15, and then the client device 2 displays the set of sign-in coordinates 145 together with the set of caregiving coordinates 144 in step S16 (see Figure 6), so as to allow the client to determine whether the caregiver signed in at the caregiving location. Similarly, in response to a request from the caregiver device 3 for the set of sign-in coordinates 145, the server 1 transmits the set of sign-in coordinates 145 further to the caregiver device 3 in step S15, and then the caregiver device 3 displays the set of sign-in coordinates 145 together with the set of caregiving coordinates 144 in step S17.

In some embodiments, once the caregiver, by operating the allowing button 321, grants permission to the server 1 to obtain a current geographic position of the caregiver device 3, the server 1 may automatically transmit a request for the set of sign-in coordinates 145 or a request for the set of sign-out coordinates 146 to the geolocation service provider 9 for the geolocation service provider 9, without enabling the caregiver device 3, to display the authorization interface 32 when the corresponding one of the sign-in button 311 and the sign-out button 312 is operated again.

On the other hand, when it is determined that the disallowing button 322 is operated on the caregiver device 3, the server 1 does not transmit the request for the set of sign-in coordinates 145 to the geolocation service provider 9. As a result, the geolocation service provider 9 will not request the sign-in position data from the caregiver device 3, and thus the server 1 will not obtain the set of sign-in coordinates 145 from the geolocation service provider 9. Further, in the case that the disallowing button 322 was operated, when it is determined later that the sign-out button 312 is operated on the caregiver device 3 or that the sign-in button 311 is operated again on the caregiver device 3, the server 1 enables the caregiver device 3 to display the authorization interface 32 again to ask the caregiver whether to allow the server 1 to obtain a current geographic position of the caregiver device 3 or not.

Steps S18 to S24 are implemented when the caregiver operates the sign-out button 312 on the caregiver device 3.

In step S18, when it is determined by the caregiver device 3 that the sign-out button 312 is operated, the caregiver device 3 records a time instant when the sign-out button 312 is being operated (a second time instant) as the sign-out time, and transmits the sign-out time to the server 1 for storage of the sign-out time in the server 1. In some embodiments, when the sign-out button 312 is operated, the caregiver device 3 further communicates with the server 1 through the APP 39 to notify the server 1 that the sign-out button 312 is operated.

In step S19, the server 1 records the sign-out time in the entry of attendance history. When determining that the sign-out button 312 of the attendance interface 31 is operated, the server 1 enables the geolocation service provider 9 to request the sign-out position data related to a current geographic position of the caregiver device 3 (i.e., a second current geographic position) from the caregiver device 3, and to transmit, to the server 1 with reference to the sign-out position data, the set of sign-out coordinates 146 corresponding to the second current geographic position of the caregiver device 3 in the GCS.

Specifically, the server 1 transmits a request for the set of sign-out coordinates 146 to the geolocation service provider 9 in step S20. Then, in step S21, the geolocation service provider 9 requests the sign-out position data from the caregiver device 3 in response to receipt of the request for the set of sign-out coordinates 146 from the server 1, and transmits to the server 1 the set of sign-out coordinates 146 with reference to the sign-out position data. Accordingly, the server 1 obtains the set of sign-out coordinates 146 from the geolocation service provider 9. In response to a request from the client device 2 for the set of sign-out coordinates 146, the server 1 transmits the set of sign-out coordinates 146 to the client device 2 in step S22, and then the client device 2 displays the set of sign-out coordinates 146 together with the set of caregiving coordinates 144 in step S23 (see Figure 6), so as to allow the client to determine whether the caregiver signed out at the caregiving location. Similarly, in response to a request from the caregiver device 3 for the set of sign-out coordinates 146, the server 1 transmits the set of sign-out coordinates 146 further to the caregiver device 3 in step S22, and then the caregiver device 3 would display the set of sign-out coordinates 146 together with the set of caregiving coordinates 144 in step S24.

In the method, each of the client device 2 and the caregiver device 3 may obtain the entry of attendance history corresponding to the appointment record 14 from the server 1, and display the entry of attendance history thus obtained (see Figure 6).

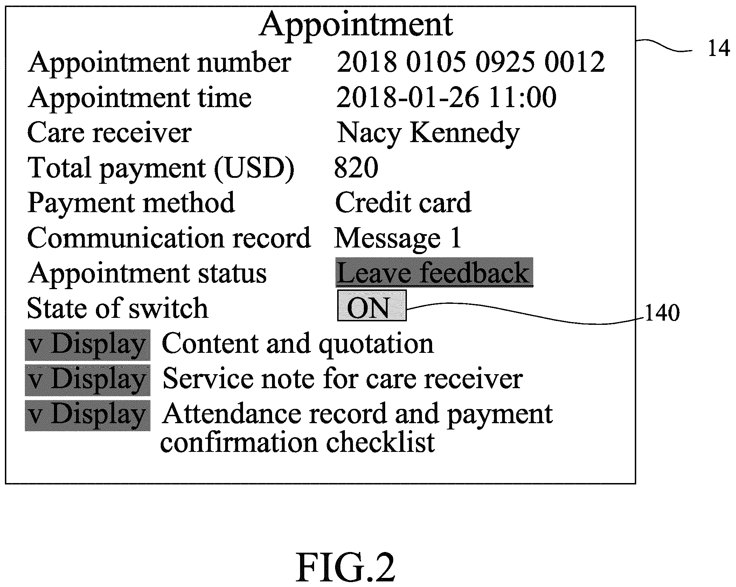

An example of the system implementing the method of Figures 9A to 9C according to some embodiments of the disclosure is described below. It should be noted that what are illustrated in Figures 2 to 8 are images of user interfaces rendered by execution of the APP 29 or the APP 39, and are presented via a display device or a touchscreen of the client device 2 or via a display device or a touchscreen of the caregiver device 3. In the following example, the client is exemplarily Anna Kennedy, the care receiver is exemplarily Nacy Kennedy, the caregiver is exemplarily Lily Carter, and the address 142 corresponding to the caregiving location is "TorstraBe 139, 10119 Berlin".

To become a caregiver, a person needs to operate the caregiver device 3 executing the APP 39, and provide required data to register as a caregiver. The required data may include personal information of the caregiver (e.g., name of the caregiver, gender of the caregiver, a phone number of the caregiver and a communication address of the caregiver), a bank account, keywords that can be used in a search for the caregiver, type(s) of care service that the caregiver is able to offer, a service area within which the caregiver is willing to go in order to provide the care service (e.g., within 500 meters, 1000 meters, 1500 meters, 2000 meters, 2500 meters, or 3000 meters of a specific location), and preferred time when the caregiver prefers to provide the care service. Thereafter, the required data provided by the caregiver is stored in the caregiver database 11 of the server 1.

Similarly, to become a client to request care service for the care receiver (e.g., an elder in the family of the client), a person needs to operate the client device 2 executing the APP 29, and provide required data to register as a client. The required data may include personal information of the client (e.g., name of the client, gender of the client, a phone number of the client and a communication address of the client), a bank account, and information related to the care receiver (e.g., name of the care receiver, gender of the care receiver, a phone number of the care receiver, and an address of a location where the care service is to be provided (i.e., the caregiving location)). Thereafter, the required data provided by the client is stored in the client database 12 of the server 1.

The client device 2 executing the APP 29 is configured to be operated by the client to make an appointment for the care service, and the server 1 is configured to store the appointment record 14, accordingly. Specifically speaking, the client device 2 executing the APP 29 is configured to be operated to access the caregiver database 11 of the server 1, and to search for caregivers that meet certain criteria inputted by the client and demanded by the care receiver (such as a specific type of care service, the care receiver's area of residence, and the time period during which the care service is required) . It is noted that since the caregiver database 11 stores the required data provided by each caregiver (e.g., the keywords, the type (s) of care service offered, the service area and the preferred time), the server 1 is able to provide a search result of those of the caregivers who have the required data meeting the criteria inputted by the client. Subsequently, the client device 2 executing the APP 29 is configured to be operated, for example by the client, to choose among the caregivers provided by the server 1 through the search, and to make an appointment for a care session with one of the caregivers thus chosen (referred to as the chosen caregiver). In response to the making of the appointment with the chosen caregiver on the client device 2, the server 1 is configured to transmit a text message to the corresponding caregiver device 3 (i.e., the caregiver device 3 corresponding to the chosen caregiver) to notify the chosen caregiver that he/she has to respond to the client by providing a quoted price for the care service within, e.g., two hours.

The caregiver device 3 executing the APP 39 is configured to be operated by the caregiver to provide the quotation or to decline the appointment requested by the client. To provide the quotation, the chosen caregiver has to operate the caregiver device 3 to provide the cost estimate (i.e., the price that the chosen caregiver will charge for providing the care session). Alternatively, the chosen caregiver may choose to decline the appointment requested by the client. In response to the provision of the quotation from the caregiver device 3, the server 1 transmits a text message to the client device 2 to notify the client that he/she has to approve the quotation and make payment by credit card or by bank transfer for the care session within, e.g., two hours based on the quotation provided by the chosen caregiver if he/she accepts the quotation. The appointment requested by the client will be automatically declined by default in case that no quotation is made by the chosen caregiver within the time limit, which is exemplarily two hours.

After the payment is completed, the appointment record 14 stored in the server 1 can be checked by the client using the client device 2 operating the APP 29 or by the caregiver using the caregiver device 3 operating the APP 39. Moreover, the server 1 is configured, when it is determined that the payment for the care session has been made, to designate the current state of the switch 140 to the ON state as shown in Figure 2, and to provide the interface data to the caregiver device 3 so as to allow the caregiver device 3 to display the attendance interface 31 (Figure 4) for allowing the caregiver to sign in and sign out. It should be noted that the current state of the switch 140 can also be designated to one of the ON state and the OFF state by operating the client device 2 executing the APP 29.

At the same time, the server 1 is configured to, when it is determined that the payment for the care service has been made, transmit the address 142 of the appointment record 14 to the digital map provider 8. Subsequently, the digital map provider 8 converts the address 142 into the set of caregiving coordinates 144, and transmits the set of caregiving coordinates 144 to the server 1 for storage of the set of caregiving coordinates 144 in the server 1. The set of caregiving coordinates 144 stored in the server 1 can be requested by each of the client device 2 and the caregiver device 3 for being displayed on the corresponding one of the client device 2 and the caregiver device 3. For example, referring to Figure 3, the address 142 and the set of caregiving coordinates 144 are displayed together on an interface of the APP 29, 39 by each of the client device 2 and the caregiver device 3. Therefore, the client or the caregiver can know the set of caregiving coordinates 144 corresponding to the address 142, i.e., the set of caregiving coordinates 144 corresponding to the caregiving location.

Referring to Figures 1, 2 and 4, the caregiver device 3 obtains the interface data that is related to the attendance interface 31 from the server 1, and automatically displays the attendance interface 31 for allowing the caregiver to sign in and sign out when it is determined that the current state of the switch 140 is in the ON state and that a current time is within the preset period, e.g., 30 minutes before the start time of the caregiving period 143.

Referring to Figures 1, 4, 5 and 6, when the caregiver operates the sign-in button 311 for the first time with respect to the appointment, which includes three care sessions as an example, to sign in for a first care session of the appointment (i.e., for the caregiving period from 8 AM to 12 PM on January 15, 2018), the server 1 transmits to the caregiver device 3 interface data that is related to the authorization interface 32 for inquiring whether the caregiver allows the server 1 to track the current position of the caregiver device 3. The caregiver may operate the allowing button 321 of the authorization interface 32 so as to allow the server 1 to obtain the set of sign-in coordinates 145 and the set of sign-out coordinates 146 corresponding to the current position of the caregiver device 3 at the time of sign-in and sign-out, or may operate the disallowing button 322 of the authorization interface 32 so as to decline the server 1 the right to obtain the set of sign-in coordinates 145 and the set of sign-out coordinates 146. It should be noted that in the event that the caregiver operates the disallowing button 322 of the authorization interface 32, the server 1 will transmit to the caregiver device 3 the interface data that is related to the authorization interface 32 again so as to inquire again whether the caregiver allows the server 1 to track the current position of the caregiver device 3 whenever later the caregiver signs in or signs out by operating the caregiver device 3. After the caregiver has operated the allowing button 321 of the authorization interface 32 to allow the server 1 to track the current position of the caregiver device 3, the server 1 no longer transmits the interface data related to the authorization interface 32 to the caregiver device 3.

When the caregiver operates the sign-in button 311, the caregiver device 3 records a first time instant when the sign-in button 311 is being operated as the sign-in time, and transmits the sign-in time to the server 1 for storage of the sign-in time in the server 1. When the caregiver operates the sign-out button 312, the caregiver device 3 records a second time instant when the sign-out button 312 is being operated as the sign-out time, and transmits the sign-out time to the server 1 for storage of the sign-out time in the server 1. The sign-in time and the sign-out time are recorded together in the entry of attendance history for the appointment record 14 of the appointment as shown in Figure 6.

For example, referring to Figure 4, for a second care session, which is on January 16, 2018, of the appointment, the scheduled sign-in time is 8 AM and the scheduled sign-out time is 12 PM. However, as shown in Figure 6, the caregiver actually signed in at 8:04 AM and signed out at 11:59 AM on January 16, 2018. In this way, the client is able to determine whether the caregiver signed in and signed out on schedule.

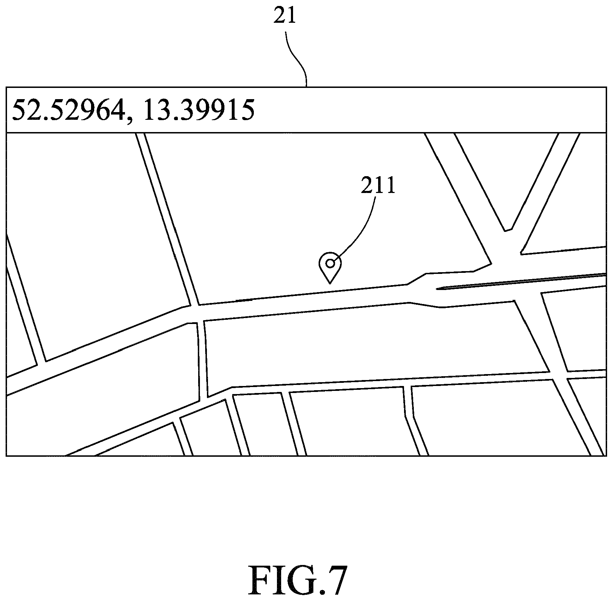

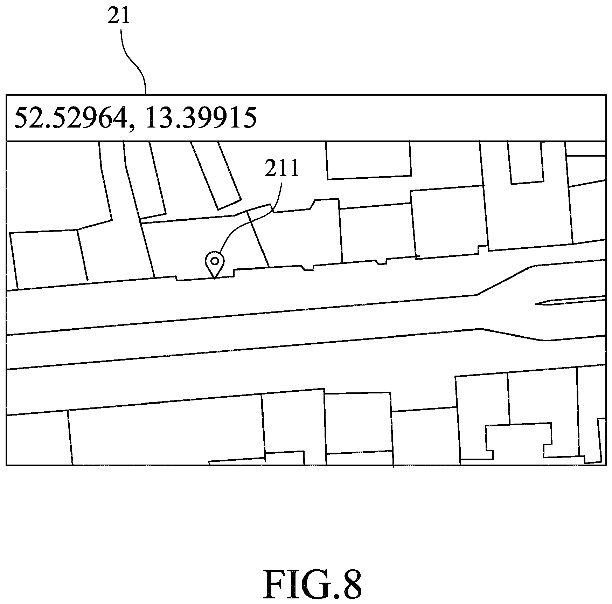

Additionally, when the caregiver operates the sign-in button 311, the server 1 enables the geolocation service provider 9 to request the sign-in position data related to a first current geographic position of the caregiver device 3 from the caregiver device 3, and to transmit to the server 1 the set of sign-in coordinates 145 corresponding to the first current geographic position of the caregiver device 3 in the GCS, with reference to the sign-in position data. When the caregiver operates the sign-out button 312, the server 1 enables the geolocation service provider 9 to request the sign-out position data related to a second current geographic position of the caregiver device 3 from the caregiver device 3, and to transmit to the server 1 the set of sign-out coordinates 146 corresponding to the second current geographic position of the caregiver device 3 in the GCS, with reference to the sign-out position data. The set of sign-in coordinates 145 and the set of sign-out coordinates 146 are recorded together in the entry of attendance history for the appointment record 14 of the appointment as shown in Figure 6. Referring to an attendance record corresponding to a sign-out time "2018/01/16 11:59" in Figure 6, the client may notice that there is an abnormally large difference between 52.52964 degrees of the latitude in the set of sign-out coordinates 146 "52.52964, 13.39915" and 52.52967 degrees of the latitude in the set of caregiving coordinates 144 "52.52967, 13.39910". To check whether the caregiver signed out within a reasonable range of the caregiving location, the client can use the set of sign-out coordinates "52.52964, 13.39915" as an input to a web mapping service (e.g., Google Maps or Apple Maps) provided by the digital map provider 8 so as to obtain a digital map 21 of an area around a target location 211 corresponding to the set of sign-out coordinates "52.52964, 13.39915" as shown in Figures 7 and 8. When a residential space of the care receiver is rather large, it may difficult for the client to determine whether the caregiver signed in or out at the caregiving location only by comparing the set of caregiving coordinates 144 with the set of sign-in or sign-out coordinates 145, 146. By inspecting the digital map 21 thus provided, the client may be able to know whether the caregiver signed out within a reasonable range of the caregiving location, regardless of how large the residence to which the caregiving location corresponds. Therefore, the system according to the disclosure enables a client to determine whether the caregiver signed in or signed out at an expected time and at an expected location.

In summary, the system for recording attendance of a caregiver according to the disclosure obtains the set of caregiving coordinates 144 by utilizing the server 1 to transmit to the digital map provider 8 the address 142 of the appointment record 14 for the care service, and obtains the sign-in coordinates 145 and sign-out coordinates 146 by utilizing the server 1 to enable the geolocation service provider 9 to request the position data from the caregiver device 3 and to transmit to the server 1 the sign-in coordinates 145 and sign-out coordinates 146, with reference to the position data. Then, the client device 2 is capable of requesting the set of caregiving coordinates 144, the set of sign-in coordinates 145 and the set of sign-out coordinates 146 from the server 1, and displaying the set of caregiving coordinates 144, the set of sign-in coordinates 145 and the set of sign-out coordinates 146 together. In this way, the client may determine whether the caregiver signed in (or signed out) at the caregiving location by comparing the set of caregiving coordinates 144 and the set of sign-in coordinates 145 (or the set of sign-out coordinates 146).

In the description above, for the purposes of explanation, numerous specific details have been set forth in order to provide a thorough understanding of the embodiment. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. A system for recording attendance of a caregiver who is expected to provide a care session of care service at a caregiving location according to an appointment made by a client, said system **characterized by**:
a server (1) configured to communicate with a digital map provider (8) and a geolocation service provider (9), to store an appointment record (14) that is related to the appointment and that has an address (142) corresponding to the caregiving location, and to transmit the address (142) to the digital map provider (8) for the digital map provider (8) to convert the address (142) into a set of caregiving coordinates (144) in a geographic coordinate system (GCS) and transmit the set of caregiving coordinates (144) to said server (1);
a client device (2) adapted to be possessed by the client, and configured to communicate with said server (1), to request from said server (1) the set of caregiving coordinates (144) corresponding to the caregiving location, and to display the set of caregiving coordinates (144); and
a caregiver device (3) adapted to be possessed by the caregiver, and configured to communicate with the geolocation service provider (9), to communicate with said server (1) to obtain from said server (1) interface data that is related to an attendance interface (31) containing a sign-in button (311), to display the attendance interface (31) based on the interface data so as to allow the caregiver to sign in with respect to the care session by operating the sign-in button (311),
wherein said server (1) is further configured to, when it is determined that the sign-in button (311) of the attendance interface (31) is operated, enable the geolocation service provider (9) to request sign-in position data related to a first current geographic position of said caregiver device (3) from said caregiver device (3), and to transmit, to said server (1) with reference to the sign-in position data, a set of sign-in coordinates (145) corresponding to the first current geographic position of said caregiver device (3) in the GCS,
wherein said client device (2) is further configured to request the set of sign-in coordinates (145) from said server (1), and to display the set of sign-in coordinates (145) together with the set of caregiving coordinates (144) so as to allow the client to determine whether the caregiver signed in at the caregiving location.

2. The system as claimed in claim 1, **characterized in that**:
the appointment record (14) stored in said server (1) further has a caregiving period (143), during which the caregiver is expected to provide the care session; and
said caregiver device (3) is configured to, when it is determined that a current time is within a preset period before a start time point of the caregiving period (143), display the attendance interface (31) for allowing the caregiver to sign in and sign out.

3. The system as claimed in claim 1, **characterized in that**:
the appointment record (14) stored in said server (1) further has a caregiving period (143), during which the caregiver is expected to provide the care session, and a current state of a switch (140);
said client device (2) is configured to be operated to switch the current state of the switch (140) between an ON state and an OFF state; and
said server (1) is configured to transmit the interface data to said caregiver device (3) so as to enable said caregiver device (3) to display the attendance interface (31) for allowing the caregiver to sign in and sign out when it is determined that the current state of the switch (140) is in the ON state and that a current time is within a preset period before a start time of the caregiving period (143).

4. The system as claimed in claim 1, **characterized in that** said client device (2) is further configured to be operated to make payment for the care session, and said server (1) is configured to, only when it is determined that the payment for the care session has been made, transmit the address (142) to the digital map provider (8) for the digital map provider (8) to convert the address (142) into the set of caregiving coordinates (144) and transmit the set of caregiving coordinates (144) to said server (1).

5. The system as claimed in claim 4, **characterized in that**:
the appointment record (14) stored in said server (1) further has a current state of a switch (140) that is switchable between an ON state and an OFF state; and
said server (1) is configured, when it is determined that the payment for the care session has been made, to designate the current state of the switch (140) to the ON state, and to transmit the interface data to said caregiver device (3) so as to enable said caregiver device (3) to display the attendance interface (31) for allowing the caregiver to sign in and sign out.

6. The system as claimed in claim 1, **characterized in that** said caregiver device (3) is configured to, when determining that the sign-in button (311) is operated, record a first time instant when the sign-inbutton (311) is being operated as a sign-in time, and transmit the sign-in time to said server (1) for storage of the sign-in time in said server (1).

7. The system as claimed in claim 6, **characterized in that**:
the attendance interface (31) further contains a sign-out button (312) allowing the caregiver to sign out with respect to the care session by operating the sign-out button (312);
said server (1) is further configured to, when it is determined that the sign-out button (312) of the attendance interface (31) is operated, enable the geolocation service provider (9) to request sign-out position data related to a second current geographic position of said caregiver device (3) from said caregiver device (3), and to transmit, to said server (1) with reference to the sign-out position data, a set of sign-out coordinates (146) corresponding to the second current geographic position of said caregiver device (3) in the GCS;
said client device (2) is further configured to request the set of sign-out coordinates (146) from said server (1), and to display the set of sign-out coordinates (146) together with the set of caregiving coordinates (144) so as to allow the client to determine whether the caregiver signed out at the caregiving location.

8. A method for recording attendance of a caregiver who is expected to provide a care session of care service at a caregiving location according to an appointment made by a client, the method to be implemented by a server (1) communicating with a client device (2), a caregiver device (3), a digital map provider (8) and a geolocation service provider (9), the server (1) storing an appointment record (14) that is related to the appointment and that has an address (142) corresponding to the caregiving location, the client device (2) being possessed by the client, the caregiver device (3) being possessed by the caregiver and capable of communicating with the geolocation service provider (9), the method **characterized by** steps of:
transmitting the address (142) to the digital map provider (8) for the digital map provider (8) to convert the address (142) into a set of caregiving coordinates (144) in a geographic coordinate system (GCS) and to transmit the set of caregiving coordinates (144) to the server (1);
in response to a request from the client device (2) for the set of caregiving coordinates (144), transmitting the set of caregiving coordinates (144) corresponding to the caregiving location to the client device (2) for the client device (2) to display the set of caregiving coordinates (144);
transmitting, to the caregiver device (3), interface data that is related to an attendance interface (31) containing a sign-in button (311) for the caregiver device (3) to display the attendance interface (31) based on the interface data so as to allow the caregiver to sign in with respect to the care session by operating the sign-in button (311);
when it is determined that the sign-in button (311) of the attendance interface (31) is operated, enabling the geolocation service provider (9) to request sign-in position data related to a first current geographic position of the caregiver device (3) from the caregiver device (3), and to transmit, to the server (1) with reference to the sign-in position data, a set of sign-in coordinates (145) corresponding to the first current geographic position of the caregiver terminal (3) in the GCS; and
in response to a request from the client device (2) for the set of sign-in coordinates (145), transmitting the set of sign-in coordinates (145) to the client device (2) for the client device (2) to display the set of sign-in coordinates (145) together with the set of caregiving coordinates (144) so as to allow the client to determine whether the caregiver signed in at the caregiving location.

9. The method as claimed in claim 8, the appointment record (14) stored in the server (1) further having a caregiving period (143), during which the caregiver is expected to provide the care session, the method to be implemented further by the caregiver device (3) and further **characterized by**:
the caregiver device (3) determining whether a current time is within a preset period before a start time point of the caregiving period (143); and
when it is determined that the current time is within the preset period before the start time point of the caregiving period (143), the caregiver device (3) displaying the attendance interface (31) for allowing the caregiver to sign in and sign out.

10. The method as claimed in claim 8, the appointment record (14) stored in the server (1) further having a caregiving period (143), during which the caregiver is expected to provide the care session, and a current state of a switch (140), the client device (2) being operable to switch the current state of the switch (140) between an ON state and an OFF state, the method to be implemented further by the caregiver device (3) and further **characterized by**:
when determining that the current state of the switch (140) is in the ON state, the server (1) transmitting the interface data to the caregiver device (3);
the caregiver device (3) determining whether a current time is within a preset period before a start time point of the caregiving period (143); and
the caregiver device (3) displaying, based on the interface data, the attendance interface (31) for allowing the caregiver to sign in and sign out when it is determined that the current time is within the preset period before the start time of the caregiving period (143) .

11. The method as claimed in claim 8, the client device (2) being operable to make payment for the care session, the method further **characterized by**:
when it is determined that the payment for the care session has been made, transmitting the address (142) to the digital map provider (8) for the digital map provider (8) to convert the address (142) into the set of caregiving coordinates (144) and transmit the set of caregiving coordinates (144) to the server (1).

12. The method as claimed in claim 11, the appointment record (14) stored in the server (1) further having a current state of a switch (140) that is switchable between an ON state and an OFF state, the method further **characterized by**:
when it is determined that the payment for the care session has been made,
designating the current state of the switch (140) to the ON state, and
transmitting the interface data to the caregiver device (3) so as to enable the caregiver device (3) to display the attendance interface (31) for allowing the caregiver to sign in and sign out.

13. The method as claimed in claim 8, to be implemented further by the caregiver device (3) and further **characterized by**:
when determining that the sign-in button (311) is operated, the caregiver device (3) recording a first time instant when the sign-in button (311) is being operated as a sign-in time, and transmitting the sign-in time to the server (1) for storage of the sign-in time in the server (1).

14. The method as claimed in claim 13, the attendance interface (31) further containing a sign-out button (312) allowing the caregiver to sign out with respect to the care session by operating the sign-out button (312), the method further **characterized by**:
when it is determined that the sign-out button (312) of the attendance interface (31) is operated, the server (1) enabling the geolocation service provider (9) to request sign-out position data related to a second current geographic position of the caregiver device (3) from the caregiver device (3), and to transmit, to the server (1) with reference to the sign-out position data, a set of sign-out coordinates (146) corresponding to the second current geographic position of the caregiver device (3) in the GCS; and
in response to a request from the client device (2) for the set of sign-out coordinates (146), the server (1) transmitting the set of sign-out coordinates (146) to the client device (2) for the client device (2) to display the set of sign-out coordinates (146) together with the set of caregiving coordinates (144) so as to allow the client to determine whether the caregiver signed out at the caregiving location.
